# EUROPEAN PATENT APPLICATION

(11) **EP 4 659 690 A1**
(43) Date of publication of application: **10.12.2025**
(21) Application number: 23919948.2
(22) Date of filing: 13.12.2023
(51) Int. Cl.: A61B 17/34

(54) **MEDICAL DEVICE**

(30) Priority: 31.01.2023 JP 2023012777
(71) Applicant: ASAHI INTECC CO., LTD., Seto-shi, Aichi, 489-0071 (JP)
(72) Inventor: HOSOMI, Ryou, Seto-shi, Aichi 489-0071 (JP); FUSEYA, Yukihiro, Seto-shi, Aichi 489-0071 (JP); KURITA, Daisuke, Seto-shi, Aichi 489-0071 (JP); SHIRAISHI, Ryuya, Seto-shi, Aichi 489-0071 (JP); MINAMI, Erina, Seto-shi, Aichi 489-0071 (JP)
(74) Representative: Winter, Brandl - Partnerschaft mbB
(86) International application number: PCT/JP2023/044730
(87) International publication number: WO 2024/161813

(57) **Abstract**

A dilator is provided that achieves both flexibility and ease of obtaining a sufficient expansion diameter.

A dilator 1 includes: a core shaft 21 including a tapered portion 23 having a diameter decreasing from a proximal end toward a distal end, and a straight portion 22, 24 adjacent to the tapered portion 23, extending along a long axis direction, and having a constant outer diameter; and a protruding portion 31 provided on an outer peripheral surface of the tapered portion 23 and the straight portion 22, 24 and spirally extending with a gap between portions adjacent to each other along the long axis direction. The protruding portion 31 includes a first protruding portion 32 located on the tapered portion 23 and a second protruding portion 33, 34 located on the straight portion 22, 24, and rigidity of the first protruding portion 32 is lower than rigidity of the second protruding portion 33, 34.

## Description

### [Technical Field]

This disclosure relates to a medical device.

### [Background Art]

There are known medical devices for expanding a hole opened in a wall of an organ such as a stomach or a liver, a constricted part in a body cavity such as a biliary duct or a pancreatic duct, or the like.

Such a medical device has a tapered portion provided in a core shaft, and when the tapered portion is pressed into a hole, the hole is expanded. Providing a spirally-arranged protruding portion on an outer peripheral surface of the tapered portion and a straight portion adjacent to the tapered portion has been proposed (for example, see Patent Literature 1).

### [Citation List]

### [Patent Literature]

[Patent Literature 1] Japanese Patent Application Laid-open No. 2014-524807

### [Summary of Invention]

### [Technical Problem]

In the medical device of Patent Literature 1, when a core shaft and a protruding portion are formed of a hard material (for example, stainless steel), a sufficient expansion diameter can be obtained. Therefore, it is considered that the medical device of Patent Literature 1 has reduced flexibility, which makes the medical device difficult to pass a bending portion such as a blood vessel. When the core shaft and the protruding portion are formed of a flexible material (for example, resin), the flexibility is improved. However, it is considered that a sufficient expansion diameter is hard to be obtained in this case.

An object of the present disclosure is to provide a medical device in which both flexibility and a sufficient expansion diameter can be obtained.

### [Solution to Problem]

In order to achieve the above object, a medical device according to an embodiment of the present disclosure is a medical device including: a core shaft including a tapered portion having a diameter decreasing from a proximal end toward a distal end, and a straight portion adjacent to the tapered portion, extending along a long axis direction, and having a constant outer diameter; and a protruding portion provided on an outer peripheral surface of the tapered portion and the straight portion and spirally extending with a gap between portions adjacent to each other along the long axis direction, in which the protruding portion includes a first protruding portion located on the tapered portion and a second protruding portion located on the straight portion, and rigidity of the first protruding portion is different from rigidity of the second protruding portion. With such a medical device, a hole having a sufficient expansion diameter can be obtained.

The first protruding portion may be formed of a resin material and the second protruding portion may be formed of a metal material. Such a medical device can be configured such that rigidity of the first protruding portion and rigidity of the second protruding portion are different from each other. For example, the rigidity of the first protruding portion can be made to be lower than the rigidity of the second protruding portion.

A transverse section of the first protruding portion and a transverse section of the second protruding portion may have the same shape. With such a medical device, a friction resistance of the first protruding portion and a puncture portion and a friction resistance of the second protruding portion and a puncture portion at the time of passing the hole can be made to be the same, and the same thrust force can be obtained in the tapered portion and the straight portion. Accordingly, in the medical device, a hole having a sufficient expansion diameter can be easily formed.

The straight portion may be provided in a proximal end side of the tapered portion. In such a medical device, the straight portion of the proximal end side enables a diameter of an expanded hole to be maintained.

The straight portion may be provided in a distal end side and the proximal end side of the tapered portion. With such a medical device, the straight portion of the distal end side facilitates insertion to a hole, and the straight portion of the proximal end side enables a diameter of an expanded hole to be maintained.

The rigidity of the first protruding portion may be lower than the rigidity of the second protruding portion. With such a medical device, both flexibility at the time of passing a bending portion and ease of obtaining a sufficient expansion diameter of a hole can be achieved.

The rigidity of the first protruding portion may be higher than the rigidity of the second protruding portion. With such a medical device, a hole having a sufficient expansion diameter can be obtained.

### [Brief Description of Drawings]

FIG. 1 is a schematic side view of a dilator according to a first embodiment.
FIG. 2 is a cross-sectional view of a first protruding portion.
FIG. 3 is a cross-sectional view of a second protruding portion.
FIG. 4 is a schematic side view of a dilator according to a second embodiment.
FIG. 5 is a schematic side view of a dilator according to a third embodiment.
FIG. 6 is a schematic side view of a dilator according to a fourth embodiment.

### [Description of Embodiment]

Hereinafter, embodiments of the present disclosure will be described with reference to the drawings. The present disclosure is not limited to the embodiments described in the drawings. The size in each drawing is a size illustrated to facilitate understanding of the embodiments, and does not correspond to the actual size.

### <First Embodiment>

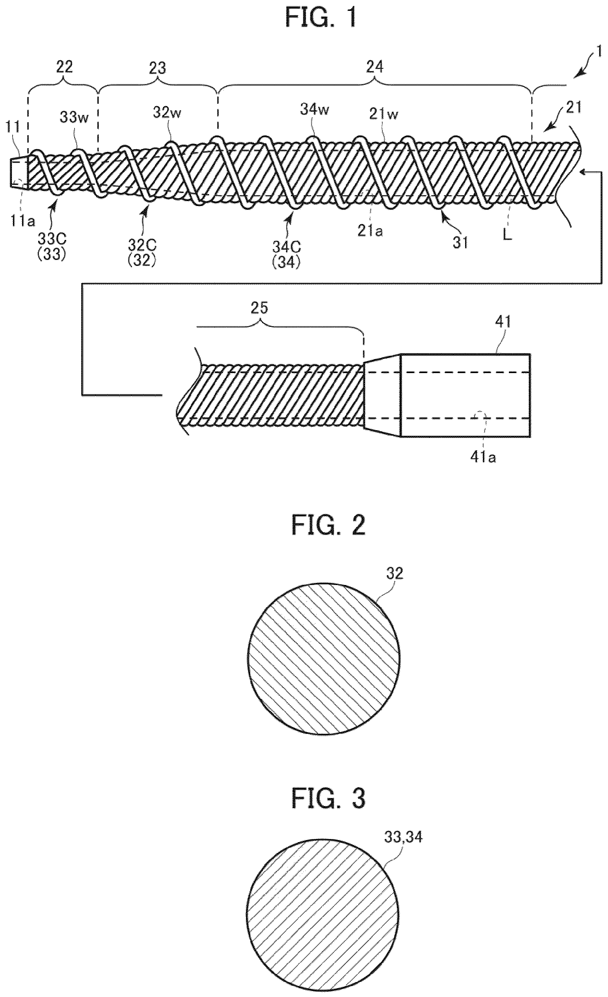

A medical device according to a first embodiment of the present disclosure will be described with reference to the drawings. FIG. 1 is a schematic side view of a dilator 1 as the medical device according to the first embodiment. In FIG. 1, a distal tip 11 side is a distal end side (far side) inserted into a body, and a grip portion 41 side is a proximal end side (hand side, near side) operated by a professional such as a doctor.

The dilator 1 includes the distal tip 11, a core shaft 21, a spirally-arranged protruding portion 31, and the grip portion 41. The distal tip 11 is located on the most distal end portion of the dilator 1. The distal tip 11 is formed by putting a brazing material (silver tin brazing material, gold tin brazing material, or the like) into the distal end of the core shaft 21. The shape of the distal tip 11 is a substantially cylindrical shape and includes an inner cavity 11a.

The core shaft 21 is a member including a tapered portion and a straight portion. The tapered portion has a diameter decreasing from a proximal end toward a distal end. The straight portion is adjacent to the tapered portion, extends along a long axis direction, and has a certain outer diameter.

A positional relationship (front and rear relationship) of the tapered portion and the straight portion in the long axis direction is not particularly limited. For example, the tapered portion and the straight portion may be arranged in this order from the distal end of the dilator, may be arranged in the order of the straight portion and the tapered portion from the distal end of the dilator, or may be arranged in the order of the straight portion, the tapered portion, and the straight portion from the distal end of the dilator.

The core shaft 21 of the present embodiment is formed of a coil body obtained by winding one or a plurality of metal strands 21w spirally around a long axis of the core shaft 21. The core shaft 21 has an inner cavity 21a penetrating from the proximal end to the distal end. A guide wire (not illustrated) or the like, for example, is inserted to the inner cavity 21a.

The core shaft 21 includes a first straight portion 22, a tapered portion 23, a second straight portion 24, and a proximal end portion 25 in this order from the distal end side. The first straight portion 22 is connected to the proximal end of the distal tip 11 and extends from the proximal end of the distal tip 11 toward the proximal end side. The tapered portion 23 is connected to the proximal end of the first straight portion 22 and has a diameter decreasing from the proximal end of the first straight portion 22 toward the distal end. The second straight portion 24 is connected to the proximal end of the tapered portion 23 and extends from the proximal end of the tapered portion 23 toward the proximal end side. The proximal end portion 25 is connected to the proximal end of the second straight portion 24 and extends from the proximal end of the second straight portion 24 toward the proximal end side. The first and second straight portions 22, 24 have a certain outer diameter.

The strand 21w forming the core shaft 21 is preferably antithrombotic, flexible, and biocompatible because the strand 21w is to be inserted into a body cavity. Examples of the material forming such a strand include a metal material of a superelastic alloy or the like such as a stainless steel and a nickel-titanium, or a resin material.

The protruding portion 31 is a portion provided on outer peripheral surfaces of the tapered portion and the straight portion. The protruding portion 31 extends spirally with gaps between portions adjacent to each other along the long axis direction. The protruding portion 31 includes a first protruding portion 32 located on the tapered portion 23 and second protruding portions 33, 34 located on the first and second straight portions 22, 24.

The first protruding portion 32 is formed of a coil body 32C obtained by winding one or a plurality of strands 32w on the outer peripheral surface of the tapered portion 23 in a direction opposite to the core shaft 21. The second protruding portions 33, 34 are respectively formed of coil bodies 33C, 34C obtained by winding one or a plurality of strands 33w, 34w on the outer peripheral surface of the first and second straight portions 22, 24 in a direction opposite to the core shaft 21.

The rigidity of the first protruding portion 32 is different from the rigidity of the second protruding portions 33, 34. The rigidity of the first protruding portion 32 is configured to be lower than the rigidity of the second protruding portions 33, 34. For example, the first protruding portion 32 is formed of a resin material and the second protruding portions 33, 34 are formed of a metal material. The metal material of the second protruding portions 33, 34 has a Young's modulus higher than that of the resin material of the first protruding portion 32. Examples of the material forming the strand 32w of the first protruding portion 32 include resin materials such as polyvinyl chloride resin, urethane resin, polyolefin resin, polyamide resin, or fluororesin. Examples of the material forming the strands 33w, 34w of the second protruding portions 33, 34 include a metal material of a superelastic alloy material or the like such as a stainless steel and a nickel-titanium. As illustrated in FIGS. 2 and 3, a transverse section of the first protruding portion 32 and a transverse section of the second protruding portions 33, 34 are substantially circular and have the same shape. The transverse section of the first protruding portion 32 refers to a cross section orthogonal to a long axis extending along the first protruding portion 32, the long axis extending spirally. This is similar for the second protruding portions 33, 34.

Examples of a joint method of the core shaft 21 and the first protruding portion 32 and the second protruding portions 33, 34 include a method of brazing using a brazing material, adhesion using an adhesive, and welding.

The grip portion 41 is a portion used by a professional for pushing the dilator 1 into a body or rotation operation. In this grip portion 41, the distal end is connected to the proximal end of the proximal end portion 25 of the core shaft 21, and includes an inner cavity 41a communicated with the inner cavity 21a of the core shaft 21. The shape of the grip portion 41 can be formed to be a shape with which the professional is easy to operate the dilator 1. The inner cavity 11a, the inner cavity 21a, and the inner cavity 41a form a lumen L. A guide wire or the like is inserted into the lumen L, for example.

Next, an example of the use mode of the dilator 1 is described as follows.

A technique will be described in which a hole is opened in a portion (for example, a wall of an organ such as a stomach or a liver, and hereinafter, also referred to as "portion to be expanded") to be treated using an introducer needle (not illustrated), and then the hole is expanded using the dilator 1. First, a doctor punctures a portion to be expanded with an introducer needle to form a hole. Next, after inserting a guide wire into an inner cavity of the introducer needle, the doctor pulls the introducer needle out.

Next, the doctor inserts a proximal end of the guide wire to the inner cavity L of the dilator 1 to insert the dilator 1 into a body. Then, the doctor causes the core shaft 21 to pass a bending portion such as a blood vessel along the guide wire without rotating while operating the grip portion 41, and delivers the distal end portion of the dilator 1 to immediately in front of the portion to be expanded. Then, the doctor inserts the distal tip 11 to the hole of the portion to be expanded, operates the grip portion 41 to rotate the core shaft 21 to push the dilator 1 to advance, so that the hole of the portion to be expanded is expanded. At this time, since the tapered portion 23 moves forward due to a screw action or the like of the spirally-arranged first protruding portion 32 caused by the rotation operation of the core shaft 21, the hole can be smoothly expanded by the tapered portion 23.

As described above, since the dilator 1 is configured such that the rigidity of the first protruding portion 32 is lower than the rigidity of the second protruding portions 33, 34, flexibility necessary for passing a bending portion can be obtained. Since the second protruding portions 33, 34 have higher rigidity than the first protruding portion 32, a sufficient expansion diameter of the hole can be obtained. As described above, according to the dilator 1 of the present embodiment, both flexibility at the time of passing a bending portion and ease of obtaining a sufficient expansion diameter can be achieved.

### <Second Embodiment>

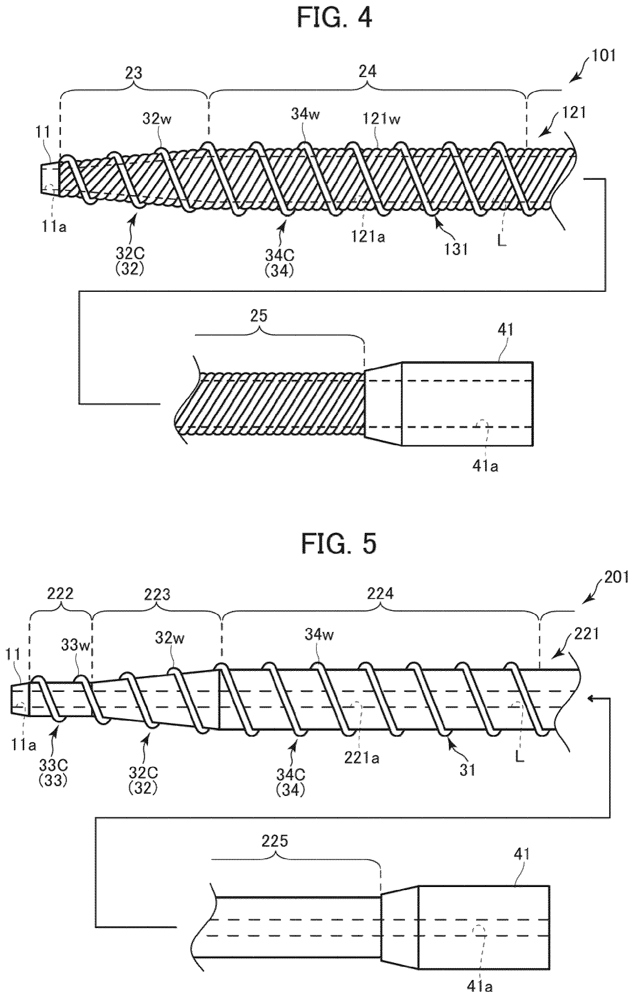

A dilator according to a second embodiment of the present disclosure will be described with reference to the drawings. FIG. 4 is a schematic side view of a dilator 101 according to the second embodiment. In FIG. 4, the distal tip 11 side is the distal end side (far side) inserted into a body, and the grip portion 41 side is the proximal end side (hand side, near side) operated by a professional such as a doctor. Since the dilator 101 of the present embodiment has basically the same structure as that of the dilator 1 of the first embodiment, the same members are denoted by the same numerals and detailed description is omitted.

The dilator 101 includes the distal tip 11, a core shaft 121, a spirally-arranged protruding portion 131, and the grip portion 41. The core shaft 121 is a member including a tapered portion and a straight portion. The tapered portion has a diameter decreasing from the proximal end toward the distal end. The straight portion is adjacent to the tapered portion, extends along a long axis direction, and has a certain outer diameter. The straight portion is provided only in a proximal end side of the tapered portion.

The core shaft 121 of the present embodiment is formed of a coil body obtained by winding one or a plurality of metal strands 121w spirally around a long axis of the core shaft 121. The core shaft 121 has an inner cavity 121a penetrating from the proximal end to the distal end. A guide wire (not illustrated) or the like, for example, is inserted to the inner cavity 121a. The inner cavity 11a, the inner cavity 121a, and the inner cavity 41a form a lumen L. The core shaft 121 includes the tapered portion 23, the straight portion 24, and the proximal end portion 25 in this order from the distal end side. The distal tip 11 is connected to the distal end of the tapered portion 23.

The protruding portion 131 is a portion provided on outer peripheral surfaces of the tapered portion and the straight portion. The protruding portion 131 extends spirally with gaps between portions adjacent to each other along the long axis direction. The protruding portion 131 includes a first protruding portion 32 located on the tapered portion 23 and a second protruding portion 34 located on the straight portion 24.

As similar to the dilator 1 of the first embodiment, according to the dilator 101 of the present embodiment, both flexibility at the time of passing a bending portion and ease of obtaining a sufficient expansion diameter can be achieved.

### <Third Embodiment>

A dilator according to a third embodiment of the present disclosure will be described with reference to the drawings. FIG. 5 is a schematic side view of a dilator 201 according to the third embodiment. In FIG. 5, the distal tip 11 side is the distal end side (far side) inserted into a body, and the grip portion 41 side is the proximal end side (hand side, near side) operated by a professional such as a doctor. In the dilator 201 of the present embodiment, the same members as those of the dilator 1 of the first embodiment are denoted by the same numerals and detailed description is omitted.

The dilator 201 includes the distal tip 11, a core shaft 221, the spirally-arranged protruding portion 31, and the grip portion 41. The core shaft 221 is a member including a tapered portion and a straight portion. The tapered portion has a diameter decreasing from the proximal end toward the distal end. The straight portion is adjacent to the tapered portion, extends along a long axis direction, and has a certain outer diameter.

The core shaft 221 of the present embodiment is formed of an integrally formed shaft having a hollow shape. The core shaft 221 has an inner cavity 221a penetrating from the proximal end to the distal end. The inner cavity 11a, the inner cavity 221a, and the inner cavity 41a form a lumen L. The core shaft 221 includes a first straight portion 222, a tapered portion 223, a second straight portion 224, and a proximal end portion 225 in this order from the distal end side.

The first straight portion 222 is connected to the proximal end of the distal tip 11 and extends from the proximal end of the distal tip 11 toward the proximal end side. The tapered portion 223 is connected to the proximal end of the first straight portion 222 and has a diameter decreasing from the proximal end of the first straight portion 222 toward the distal end. The second straight portion 224 is connected to the proximal end of the tapered portion 223 and extends from the proximal end of the tapered portion 223 toward the proximal end side. The proximal end portion 225 is connected to the proximal end of the second straight portion 224 and extends from the proximal end of the second straight portion 224 toward the proximal end side. The first and second straight portions 222, 224 have a certain outer diameter.

The material forming the core shaft 221 is preferably antithrombotic, flexible, and biocompatible because the material is to be inserted into a body cavity. Examples of such a material include resin materials such as a polyamide resin, a polyolefin resin, a polyester resin, a polyurethane resin, a silicone resin, and a fluorocarbon resin, metal materials such as stainless steel and superelastic alloys (a nickel-titanium alloy), and the like.

The protruding portion 31 is provided on the outer peripheral surfaces of the tapered portion and the straight portion. The protruding portion 31 includes a first protruding portion 32 located on the tapered portion 223 and second protruding portions 33, 34 located on the first and second straight portions 222, 224.

Examples of a joint method of the core shaft 221 and the first protruding portion 32 and the second protruding portions 33, 34 include a method of brazing using a brazing material, adhesion using an adhesive, and welding.

As similar to the dilator 1 of the first embodiment, according to the dilator 201 of the present embodiment, both flexibility at the time of passing a bending portion and ease of obtaining a sufficient expansion diameter can be achieved.

### <Fourth Embodiment>

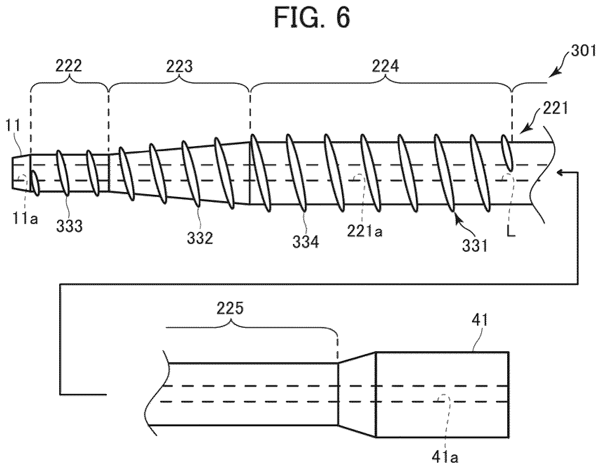

A dilator according to a fourth embodiment of the present disclosure will be described with reference to the drawings. FIG. 6 is a schematic side view of a dilator 301 according to the fourth embodiment. In FIG. 6, the distal tip 11 side is the distal end side (far side) inserted into a body, and the grip portion 41 side is the proximal end side (hand side, near side) operated by a professional such as a doctor. In the dilator 301 of the present embodiment, the same members as those of the dilator 201 of the third embodiment are denoted by the same numerals and detailed description is omitted.

The dilator 301 includes the distal tip 11, the core shaft 221, a spirally-arranged protruding portion 331, and the grip portion 41.

The protruding portion 331 is a portion provided on the outer peripheral surface of the tapered portion, or the tapered portion and the straight portion. The protruding portion 331 has gaps between portions adjacent to each other along the long axis direction. Specifically, the protruding portion 331 can be configured by a single-thread or multi-thread protrusion portion protruding from the outer peripheral surface of the tapered portion and the straight portion to the radially outward side and being continuous or intermittent in the long axis direction. In the present embodiment, the protruding portion 331 and the core shaft 221 are integrally formed. The protruding portion 331 includes a first protruding portion 332 located on the tapered portion 223 and second protruding portions 333, 334 located on the first and second straight portions 222, 224.

Examples of a material forming the core shaft 221 and the protruding portion 331 in the present embodiment include metal materials such as stainless steel and superelastic alloys (a nickel-titanium alloy) and the like. The protruding portion 331 is formed integrally with the core shaft 221 by molding or the like. The rigidity of the first protruding portion 332 is different from the rigidity of the second protruding portions 333, 334. By performing heat treatment or the like on the first protruding portion 332, the rigidity of the first protruding portion 332 is configured to be lower than the rigidity of the second protruding portions 333, 334.

As similar to the dilator 1 of the first embodiment, according to the dilator 301 of the present embodiment, both flexibility at the time of passing a bending portion and ease of obtaining a sufficient expansion diameter can be achieved.

The embodiments of the present disclosure have been described above. The disclosure is not limited to the configuration of the above-described embodiments, but is defined by the terms of the claims and is intended to include any modifications within the scope and meaning equivalent to the terms of the claims. For example, a part of the configurations of the above embodiment may be deleted or replaced by another configuration, or another configuration may be added to the configuration of the above embodiment.

In the third and fourth embodiments, for example, the core shaft 221 may not include the first straight portion 222. In this case, the tapered portion 223 is connected to the proximal end of the distal tip 11.

In the above-described embodiments, the material forming the first protruding portion 32 is a resin material, and the material forming the second protruding portions 33, 34 is a metal material. However, the materials forming the first protruding portion 32 and the second protruding portions 33, 34 may be a resin material or a metal material as long as the rigidity of the first protruding portion 32 is lower than the rigidity of the second protruding portions 33, 34. The rigidity of the first protruding portions 32, 332 is configured to be lower than the rigidity of the second protruding portions 33, 34, 333, 334. However, the rigidity of the first protruding portions 32, 332 may be configured to be higher than the rigidity of the second protruding portions 33, 34, 333, 334. According to the dilator having the configuration, a sufficient expansion diameter can be obtained.

## Claims

1. A medical device (1, 101, 201, 301) comprising:
a core shaft (21, 121, 221) including a tapered portion (23, 223) having a diameter decreasing from a proximal end toward a distal end, and a straight portion (22, 24, 222, 224) adjacent to the tapered portion (23, 223), extending along a long axis direction, and having a constant outer diameter; and
a protruding portion (31, 131, 331) provided on outer peripheral surfaces of the tapered portion and the straight portion (22, 24, 222, 224) and spirally extending with a gap between portions adjacent to each other along the long axis direction,
wherein the protruding portion (31, 131, 331) includes a first protruding portion (32, 332) located on the tapered portion (23, 223) and a second protruding portion (33, 34, 333, 334) located on the straight portion (22, 24, 222, 224), and rigidity of the first protruding portion (32, 332) is different from rigidity of the second protruding portion (33, 34, 333, 334).

2. The medical device (1, 101, 201, 301) according to claim 1, wherein the first protruding portion (32, 332) is formed of a resin material, and the second protruding portion (33, 34, 333, 334) is formed of a metal material.

3. The medical device according to claim 1 or 2, wherein a transverse section of the first protruding portion (32) and a transverse section of the second protruding portion (33, 34) have the same shape.

4. The medical device (1, 101, 201, 301) according to any one of claims 1 to 3, wherein the straight portion (24, 224) is provided in a proximal end side of the tapered portion (23, 223).

5. The medical device (1, 201, 301) according to any one of claims 1 to 4, wherein the straight portion (22, 24, 222, 224) is provided in a distal end side and the proximal end side of the tapered portion (23, 223).

6. The medical device (1, 101, 201, 301) according to any one of claims 1 to 5, wherein the rigidity of the first protruding portion (32, 332) is lower than the rigidity of the second protruding portion (33, 34, 333, 334).

7. The medical device (1, 101, 201, 301) according to any one of claims 1 to 5, wherein the rigidity of the first protruding portion (32, 332) is higher than the rigidity of the second protruding portion (33, 34, 333, 334).
